(19) Europäisches Patentamt
European Patent Office
Office européen des brevets

(11) Publication number: **0 180 416 A2**

(12) # EUROPEAN PATENT APPLICATION

(21) Application number: 85307685.9

(22) Date of filing: 24.10.85

(51) Int. Cl.⁴: C 07 C 93/20, A 61 K 31/24, C 07 D 295/08

(30) Priority: 30.10.84 JP 521/84U
25.09.85 JP 213579/85

(43) Date of publication of application: 07.05.86 Bulletin 86/19

(84) Designated Contracting States: AT BE CH DE FR GB IT LI LU NL SE

(71) Applicant: Takeda Chemical Industries, Ltd., 27, Doshomachi 2-chome Higashi-ku, Osaka-shi Osaka, 541 (JP)

(72) Inventor: Nohara, Akira, 15-12, Oharano-Kamisatotorimicho, Nishikyo-ku Kyoto 610-11 (JP)
Inventor: Maki, Yoshitaka, 5-17, Oharano-Kamisatotorimicho, Nishikyo-ku Kyoto 610-11 (JP)

(74) Representative: Laredo, Jack Joseph et al, Elkington and Fife High Holborn House 52/54 High Holborn, London, WC1V 6SH (GB)

(54) **Benzoic acid analogues and their production.**

(57) A benzoic acid derivative of the formula:

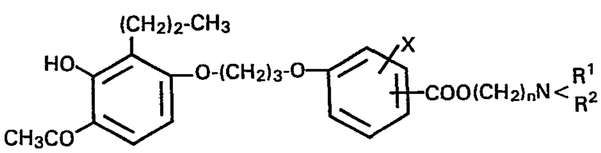

wherein $R^1$ and $R^2$ independently stand for a hydrogen atom, a lower alkyl group, or, they form, together with the adjacent nitrogen atom, a 5- or 6-membered heterocyclic ring, n denotes an integer or 2 to 4 and X stands for a halogen atom or a salt thereof, has excellent antiasthmatic and antiinflammatory activities and therefore is useful as an antiasthmatic or antiinflammatory agent for a mammalian animal.

EP 0 180 416 A2

ACTORUM AG

# Benzoic Acid Analogues and Their Production

This invention relates to a benzoic acid derivative or a salt thereof exhibiting an antagonistic action on the slow reacting substance of anaphylaxis (SRS-A), which is a group of chemical mediators, which induces a contraction of bronchial and other smooth muscles, and being useful as , among others, an antiasthmatic agent, and to a method for producing them.

As compounds exhibiting an antagonistic action on the slow reacting substance of anaphylaxis, which is a group of chemical mediators, which induces a contraction of bronchial smooth muscle, there may be mentioned those disclosed in British Patent Application Publication No. 1,384,530, European Patent Application Publication No. 28,063 and European Patent Application Publication No.80,371.

However, an antagonistic action of those compounds on SRS-A specifically disclosed in working examples of the British Patent Application Publication No. 1,384,530 and European Patent Application Publication No. 28,063 can hardly be satisfactory and an improvement of the action has been desired. As to the compound disclosed in European Patent Application Publication No. 80,371, an improvement in oral absorbability and an increase in the durability of action have been desired.

The present inventors have diligently made research work aiming at obtaining a compound which has a satisfactory antagonistic action on SRS-A, and found that a certain group of benzoic acid derivatives meet the purpose. This finding was followed by further studies, on which the present invention has been predicated.

Thus, the present invention relates to

(1) a benzoic acid derivative (I) of the formula;

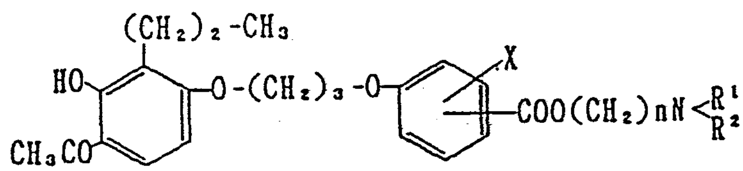 (I)

wherein $R^1$ and $R^2$ independently stand for a hydrogen atom, a lower alkyl group, or, they form, together with the adjacent nitrogen atom, a 5- or 6-membered heterocyclic ring, n denotes an integer of 2 to 4 and X stands for a halogen atom or a salt thereof,

(2) a method for producing a benzoic acid, derivative (I) or a salt thereof, which comprises allowing a reactive derivative of a compound (II) of the formula:

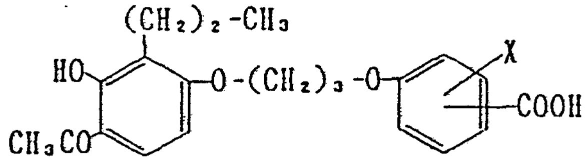 (II)

wherein X stands for a halogen to react with a reactive derivative (IV) of the formula;

$$HO-(CH_2)nN<{R^1 \atop R^2} \qquad (IV)$$

wherein $R^1$, $R^2$ and n are of the same meaning as defined above , and

(3) a method for producing a compound (I) or a salt thereof, which comprises allowing a compound (II) or a salt thereof to react with a reactive derivative of the formula;

$$Z-(CH_2)nN<{R^1 \atop R^2} \qquad (V)$$

wherein Z stands for a leaving group when the compound is esterified, and $R^1$, $R^2$ and n are of the same meaning as defined above .

The lower alkyl group representable by the symbols $R^1$ and $R^2$ in the above formulae is preferably a $C_{1-3}$ one, which is exemplified by methyl, ethyl, n-propyl and iso-propyl.

The 5- or 6-membered heterocyclic group formed with $R^1$ and $R^2$ together with the adjacent nitrogen atom is preferably such one as contains one oxygen atom or 1 to 2 nitrogen atoms, which is specifically exemplified by morpholino, piperazino, piperidino or pyrrolidino.

The above-mentioned heterocyclic groups may have a substituent which is exemplified by a $C_{1-3}$ lower alkyl (e.g. methyl, ethyl or n-propyl). In the above formulae, the halogen representable by the symbol X is exemplified by bromine, chlorine, fluorine or iodine.

It is preferable that the X in the compounds (I) and (II) is located at the ortho-position while the -COOH group and $-COO(CH_2)_nN\begin{smallmatrix}R^1\\R^2\end{smallmatrix}$ is located at the para-position.

The reactive derivatives of the compound (II) is exemplified by an acid halogenide. As halogen in the acid halogenide are mentioned chlorine, bromine, iodine, etc.

The group representable by the symbol Z, which leaves at the esterification, is exemplified by halogen (e.g. chlorine, bromine, iodine etc.), p-toluenesulfonyloxy, methanesulfonyloxy, hydroxyl group.

In case the reactive derivatives of the compound (II) are acid halogenides, they can be produced by a halogenating agent to act on a compound (II) or a salt thereof. The halogenating agent for that purpose is exemplified by thionyl chloride, phosphoryl chloride, thionyl bromide, phosphorus pentachloride, phosphorus trichloride, phosphorus oxychloride and phosphorus tribromide. A compound (II) is allowed to react with the halogenating agent in a solvent, for example,

chloroform, dichloromethane, tetrachloroethane, tetrahydrofuran or dioxane at about 80 to 120°C for about 0.5 to 12 hours.

For the reaction of the above-mentioned acid halogenide (III) with the reactive derivative (IV), about 1 to 3 equivalents of the compound (IV) is used relative to 1 equivalent of the acid halogenide (III). The solvent to be employed is exemplified by acetone, chloroform, dichloromethane, dimethylformamide, tetrahydrofuran and dioxane. The reaction is conducted preferably in the presence of a base such as triethylamine, pyridine, 4-dimethylaminopyridine or dimethylaniline. The reaction temperature ranges from about 15 to 80°C, and the reaction time is about 1 to 10 hours.

In the process of allowing a compound (II) or a salt thereof to react with a compound (V), when the esterification is conducted by using the compound wherein Z is halogen, p-toluenesulfonyloxy or methanesulfonyloxy group, a salt of compound (II) such as sodium, potassium, silver or tributylammonium salt is employed, and the amount of compound (V) to be used is about 1 to 10 equivalents relative to one equivalent of the compound (II). The reaction is preferably conducted in the presence of a base. The base is exemplified by triethylamine, pyridine, dimethylaniline and 4-dimethylaminopyridine. For the reaction is employed a solvent such as dimethylformamide, hexamethylphosphoric triamide,

acetone, methyl ethyl ketone, tetrahydrofuran or dioxane. The reaction temperature ranges from about 100°C to about 150°C, and the reaction time is about 1 to 6 hours.

The esterification between a compound (II) and a compound (IV) is conducted by a *per se* conventional method. For example, relative to one equivalent of a compound (II), about 1 to 3 equivalents of a compound (IV) is employed, and, as a catalyst, is used an excess amount of e.g. sulfuric acid, p-toluenesulfonic acid, hydrochloric acid, boron trifluoride etherate and triethyloxonium fluoroborate-diisopropylethylamine. The reaction temperature is usually within the range of about 50 to 120°C. The reaction is conducted in the absence or presence of a solvent such as toluene, dichloroethane and tetrahydrofuran.

The compound obtained by the above method can be separated from the reaction mixture and purified by a *per se* known process e.g. chromatography or recrystallization.

Preferable salts of the starting compounds as well as the end products in the method of this invention, which are pharmacologically acceptable ones, are exemplified by an alkali metal salt such as sodium salt or potassium salt, and an inorganic or organic acid salt such as hydrochloride, sulfate, phosphate, fumarate, maleate or oxalate.

The starting compound (II) of the method of this

invention can be prepared by the method described in the European Patent Application Publication No. 80,371 or by a method analogous thereto.

The compound (I) according to this invention exhibits a remarkable antagonistic action on the slow reacting substance of anaphylaxis (SRS-A), which is a group of chemical mediators, which induces a contraction of bronchial and other smooth muscles.

SRS-A is produced by various stimuli such as immune reactions and has been considered to be a potent mediator of bronchospasm in immediate allergies such as allergic asthma. SRS-A consists of leukotriene C(LTC), leukotriene D(LTD), etc.,
and it is known that LTD and LTC are substantially equivalent in activity on the human bronchial muscle and that LTD is superior to LTC in constrictive effect on the guinea pig ileum [S.E. Dahlen et al., Nature 288, 484 (1980); R.A. Lewis et al., Biochemical and Biophysical Research Communications 96, 271 (1980)]. The antagonistic effect of drugs against SRS-A can be investigated using the guinea pig ileum [R.A. Appleton et al., Journal of Medicinal Chemistry 20, 371 (1977)] and since SRS-A is a mixture of LTC, LTD, etc. and the ratio thereof is indefinite, it is desirable to use a synthetic SRS-A in the investigation of antagonistic activity.

The present inventors studied the antagonistic

action of compound (I) against SRS-A using a synthetic $LTD_4$ in the following manner, and found that, against the bronchoconstriction in guinea pigs due to an intravenous administration of synthetic leukotriene $D_4$ ($LTD_4$), compound (I)-1 to (I)-4 when administered orally one hour before $LTD_4$ dosing displayed a remarkable inhibitory effect superior to the control compound (A).

(1) Test method

Guinea pigs of Hartley strain, both male and female, with body weights about 400 g were assigned to groups of 6 to 10 individuals, and the bronchoconstriction due to $LTD_4$ was measured according to the method of Konzett-Rössler [Konzett, H. and Rössler, R.: Naunyn-Schmiedebergs Archiv für Experimentelle Pathologie und Pharmakologie 195, 71-74 (1940)]. Each guinea pig was fixed in a supine position under urethane anesthesia (1.5 g/kg, intraperitoneal) and the trachea was incised and connected to an artificial respiration apparatus, Rodent Respirator Model 680 [Harvard Apparatus Company, U.S.A.] via a cannula. The branch tube of this tracheal cannula was connected to a Bronchospasm Transducer Model 7020 [Ugobasil Biological Researach Apparatus, Italy]. Under the conditions of 4 to 7 ml of air per stroke, 70 strokes per minute and a lung loading pressure of 10 cm $H_2O$, the volume of overflowing air was recorded on a Rectigraph-8S (Sanei Sokki Ltd., Japan) via a transducer.

After administration of gallamine•triethiodide (1 mg/kg, i.v.), a solution of $LTD_4$ in physiological saline (10 μg/kg) was intravenously administered and the bronchoconstriction elicited thereby was recorded for 15 minutes. The compound was used as suspended in a 5% solution of gum arabic or dissolved in water, and administered orally in a volume of 0.2 ml per 100 g body weight one hour before $LTD_4$ loading. $LTD_4$ was administered through a cannula inserted into the jugular vein. $LTD_4$ was used as dissolved in physiological saline, which was taken from a stock stored in methanol (1 mg/1 mℓ methanol) at -70°C.

(2) Result

| Compound (I) | $-(CH_2)_nN<^{R^1}_{R^2}$ | X | $ID_{50}$(p.o.)*1 ($\mu$mol/Kg) | Example of production method |
|---|---|---|---|---|
| (I)-1*2 | $-(CH_2)_2N(CH_3)_2$ | Br | 49 | Example 1(1) |
| (I)-2 | $-(CH_2)_2$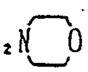 | Br | 41 | Example 2(2) |
| (I)-3*3 | $-(CH_2)_2$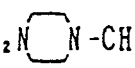$-CH_3$ | Br | 49 | Example 1(2) |
| (I)-4 | $-(CH_2)_3N(CH_3)_2$ | Br | 52 | Example 2(1) |
| Control compound (A)*4 | II | Br | 146 | |

*1 $ID_{50}$ (50% inhibitory dose): Each value was calculated from the relation between dosage and the inhibition rate of the overflow volume (in percentage) from the respiratory tract at the time when the response was maximal, i.e. 30

seconds after the administration of $LTD_4$.

*2 Hydrochloride•monohydrate

*3 Dihydrochloride•monohydrate

*4 Control compound (A): The compound disclosed in the specification of European Patent Application Publication No. 80,371, shown by the formula;

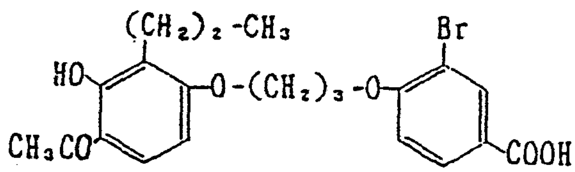

The acute toxicity of compound (I)-2 and (I)-4 in mice was found to be as follows.

(1) Method

Five 5-week-old male mice of Jcl:ICR strain, weighing about 30 g each, were used. Compound (I)-2 or (I)-4 was suspended in a 5% solution of gum arabic and administered orally at the level of 0.2 ml per 10 grams body weight.

(2) Results

The oral administration of compound (I)-2 or (I)-4 in a dose of 2000 mg/kg caused no symptoms that could be attributable to compound (I)-2 or (I)-4. Autopsy after 7 days did not reveal any abnormalities.

From the foregoing, the toxicity of compound (I) is considered to be extremely low.

It will thus be apparent that the compound (I) according to this invention is useful in the treatment of diseases due to SRS-A, such as asthma, hay fever, chronic bronchitis, allergic diseases of the eye, allergic diseases of the stomach and intestines, cardiovascular disturbances, allergic dermatitis and other inflammatory diseases. For example, as an antiasthmatic or antiinflammatory drug, the compound (I) or salts thereof can be administered orally or parenterally to mammalian animals (e.g. mouse, rat, guinea pig, man) in a daily dose of about 1 to 20 mg/kg.

For oral administration, the compound (I) or salts thereof can be formulated with a pharmaceutically acceptable carrier, excipient or diluent (e.g. lactose, starch, cellulose derivatives, stearic acid, magnesium stearate, sucrose, gelatin, gum arabic) and processed into such dosage forms as tablets, capsules, granules, troches, liquid, syrup, etc. For parenteral administration, the compound (I) or salts thereof can be formulated with pharmacologically acceptable vehicles, excipients or diluents (e.g. white petrolatum, hydrophilic ointment bases, oleaginous bases, glyceride, polyethylene glycol, etc.) and processed into ointments, suppositories, aerosols, inhalants, injections, etc. These dosage forms may be produced by the established pharmaceutical procedures.

The following Reference Examples and Examples illustrate the present invention in more detail.

Reference Example 1

By a procedure analogous to that described in the specification of European Patent Application Publication No. 80,371, the following compounds were prepared.

(1) 3-[3-(4-acetyl-3-hydroxy-2-propylphenoxy)propoxy]-4-bromobenzoic acid methyl ester, m.p. 108-110°C (Recrystallization solvent: acetone-ether).

(2) 3-[3-(4-acetyl-3-hydroxy-2-propylphenoxy)propoxy]-4-bromobenzoic acid, m.p. 146-149°C (Recrystallization solvent: aqueous alcohol).

Reference Example 2

(1) A mixture of 3-fluoro-4-hydroxybenzoic acid (1.2 g), ethanol (20 ml) and concentrated sulfuric acid ( 1 ml) was refluxed for 13 hours, then concentrated and extracted with chloroform. The extract was washed with aqueous sodium hydrogen carbonate solution, dried and the solvent was distilled off, whereby crystals of ethyl 3-fluoro-4-hydroxybenzoate (1.3 g) were obtained. m.p. 79-80°C.

(2) A mixture of ethyl 3-fluoro-4-hydroxybenzoate (600 mg), 4-(3-chloropropoxy)-2-hydroxy-3-propylacetophenone (1.15 g), potassium carbonate (450 mg), potassium iodide (550 mg) and dimethylformamide (2 ml) was stirred well for 4.5 hours at 80 to 90°C. To the reaction mixture was added ethyl acetate, and the insolubles were removed by filtration. The filtrate was subjected to concentration and then the residue was recrystallized from methanol to give crystals of ethyl 4-[3-(4-acetyl-3-hydroxy-2-propylphenoxy)propoxy]-3-fluorobenzoate (1.3 g).
m.p. 87-88°C.

(3) A mixture of ethyl 4-[3-(4-acetyl-3-hydroxy-2-propylphenoxy)-propoxy]-3-fluorobenzoate (1.3g), ethanol (8ml) and sodium hydroxide (1g) in water (5ml) was refluxed for 40 minutes. Ethanol was distilled off from the reaction mixture. The residue was dissolved in water, and the solution was acidified with dilute hydrochloric acid to give crystals. Recrystallization from ethanol gave crystals (1.06g) of 4-[3-(4-acetyl-3-hydroxy-2-propylphenoxy)-propoxy]-3-fluorobenzoic acid. m.p. 165-166°C.

Example 1

(1) A mixture of 4-[3-(4-acetyl-3-hydroxy-2-propylphenoxy)propoxy]-3-bromobenzoic acid (2 g), chloroform (20 ml) and thionyl chloride (6 ml) was refluxed for 30 minutes, and was then concentrated. The concentrate was dissolved in acetone (40 ml). To the solution was added N,N-dimethylethanolamine (400 mg), to which was added dropwise triethylamine (2 ml), and the mixture was stirred at room temperature. Two hours later, resulting precipitates were removed by filtration. The filtrate was concentrated, and the concentrate was chromatographed on a column of silica-gel. Elution was conducted with ethylacetate. From the eluate was evaporated off the solvent, and the residual oily substance was dissolved

in ether. To the solution was added etheric hydrochloride, and then the ether was evaporated. The residue was crystallized from isopropanol, and the crystals were collected by filtration, which were washed with isopropylether, followed by drying to give crystals (2.1 g) of 2-(N,N-dimethylamino)ethyl 4-[3-(4-acetyl-3-hydroxy-2-propylphenoxy)propoxy]-3-bromobenzoate•hydrochloride•monohydrate, m.p. 38-40°C (hygroscopic).

(2) By a procedure similar to the above was prepared 2-(4-methyl-1-piperazinyl)ethyl 4-[3-(4-acetyl-3-hydroxy-2-propylphenoxy)propoxy]-3-bromobenzoate•dihydrochloride•monohydrate (recrystallized from isopropanol), m.p. 142-144°C.

(3) By a procedure similar to the above was prepared 2-morpholinoethyl 3-[3-(4-acetyl-3-hydroxy-2-propylphenoxy)propoxy]-4-bromobenzoate•mono-oxalate, m.p. 145-148°C.

Example 2

(1) A mixture of 4-[3-(4-acetyl-3-hydroxy-2-propylphenoxy)propoxy]-3-bromobenzoic acid (2 g), chloroform (20 ml) and thionyl chloride (5 ml) was refluxed for 30 minutes. Chloroform was evaporated off, and the residue was dissolved in acetone (40 ml). To the solution were added 3-(N,N-dimethylamino)-1-propanol (500 mg), then triethylamine (4 ml), and the mixture was stirred at room temperature for two hours.

Resulting precipitates were filtered off, and the filtrate was concentrated, and the concentrate was chromatographed on a column of silica gel. Elution was conducted with ethyl acetate then with ethyl acetate-triethylamine (10:1). From the eluate was removed the solvent by evaporation. The residue was recrystallized from hexane to give crystals of 3-(N,N-dimethylamino)propyl 4-[3-(4-acetyl-3-hydroxy-2-propylphenoxy)propoxy]-3-bromobenzoate (1.8 g), m.p. 71-72°C.

(2) By a procedure similar to the above was prepared crystals of 2-morpholinoethyl 4-[3-(4-acetyl-3-hydroxy-2-propylphenoxy)propoxy]-3-bromobenzoate (recrystallized from hexane-chloroform), m.p. 98-99°C.

(3) By a procedure similar to the above (1) was prepared 2-(pyrrolidin-1-yl)ethyl 4-[3-(4-acetyl-3-hydroxy-2-propylphenoxy)propoxy]-3-bromobenzoate, m.p. 79-80°C.

(4) By a procedure similar to the above (1) was prepared 4-dimethylaminobutyl 4-[3-(4-acetyl-3-hydroxy-2-propylphenoxy)propoxy]-3-bromobenzoate, m.p. 69-70°C.

Example 3

A mixture of 4-[3-(4-acetyl-3-hydroxy-2-propylphenoxy)propoxy]-3-bromobenzoic acid (1 g), dimethylformamide (20 ml), triethylamine (5 ml) and dimethylaminoethylchloride hydrochloride (1 g) was heated at 150°C for one hour. The solvent was evaporated off, and the residue was dissolved in ethyl acetate.

The solution was washed with an aqueous solution of sodium carbonate, which was dried on sodium sulfate. The resulting solution was chromatographed on a column of silica gel. Elution was conducted with ethyl acetate, followed by converting the eluted substance into a hydrochloride by a procedure similar to that in Example 2 to give crystals of 2-dimethylaminoethyl 4-[3-(4-acetyl-3-hydroxy-2-propylphenoxy)-propoxy]-3-bromobenzoate•hydrochloride•monohydrate, m.p. 38-40°C (hygroscopic).

Example 4

A mixture of 4-[3-(4-acetyl-3-hydroxy-2-propylphenoxy)-propoxy]-3-chlorobenzoic acid (1 g), chloroform (10 ml) and thionyl chloride (3 ml) was refluxed for one hour. The solvent was evaporated off. Further, volatile acid was removed using sodium hydroxide under reduced pressure. The resulting acid chloride was dissolved in acetone (20 ml). To the solution were added 3-dimethylaminopropanol (300 mg) and triethylamine (2 ml). The mixture was stirred at room temperature for one hour. Resulting precipitates were filtered off, and the filtrate was concentrated. The concentrate was purified by means of a silica-gel column chromatography (ethyl acetate). The resulting oily substance was crystallized from hexane to give 1.03 g of 3-(dimethylamino)-1-propyl 4-[3-(4-acetyl-3-hydroxy-2-propylphenoxy)propoxy]-3-chlorobenzoate as crystals, m.p. 72-73°C.

Example 5

A solution of 4-[3-(4-acetyl-3-hydroxy-2-propylphenoxy)propoxy]-3-fluorobenzoic acid (220 mg) in chloroform (2 ml) and thionyl chloride (0.4 ml) was refluxed for 20 minutes, then the solvent was removed under reduced pressure. To the residue was added toluene, and then the solvent was removed under reduced pressure. To the residue thus obtained was added acetone (5 ml), 3-dimethylaminopropanol (70 mg) and triethylamine (0.25 ml), and the mixture was refluxed for 0.5 hour. The precipitated colorless crystals were removed by filtration, and the filtrate was concentrated. The residue was dissolved in chloroform, and the solution was washed once with a saturated sodium hydrogen carbonate, and dried with sodium sulfate. The solvent was removed by evaporation to the cooled residue and hexane was added to give colorless crystals of 3-dimethylaminopropyl 4-[3-(4-acetyl-3-hydroxy-2-propylphenoxy)-propoxy]-3-fluorobenzoate (140 mg).

m.p. 55-56°C.

CLAIMS

1. A compound of the formula:

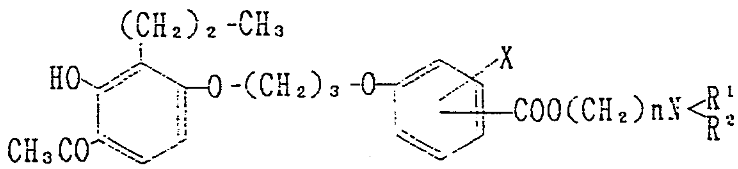

wherein $R^1$ and $R^2$ independently stand for a hydrogen atom, a lower alkyl group, or, they form, together with the adjacent nitrogen atom, a 5- or 6-membered heterocyclic ring, n denotes an integer of 2 to 4 and X stands for a halogen atom or a salt thereof.

2. A compound as claimed in Claim 1, wherein $R^1$ and $R^2$ are lower alkyl groups.

3. A compound as claimed in Claim 1, wherein $R^1$ and $R^2$ form, together with the adjacent nitrogen atom, a 6-membered heterocyclic ring.

4. A compound as claimed in Claim 1, wherein n is 2.

5. A compound as claimed in Claim 1, wherein n is 3.

6. A compound as claimed in Claim 1, wherein X is bromine.

7. A compound as claimed in Claim 1, wherein the compound is 3-(N,N-dimethylamino)propyl 4-[3-(4-acetyl-3-hydroxy-2-propylphenoxy)propoxy]-3-bromobenzoate.

8. A compound as claimed in Claim 1, wherein the compound is 2-morpholinoethyl 4-[3-(4-acetyl-3-hydroxy-2-propylphenoxy)propoxy]-3-bromobenzoate.

9. A method for producing a benzoic acid derivative of the formula:

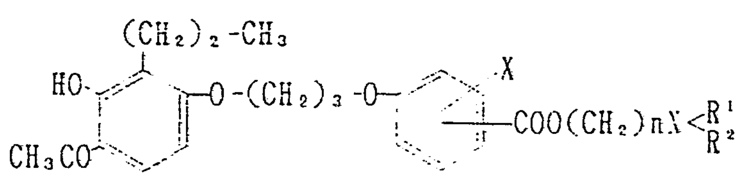

wherein $R^1$ and $R^2$ independently stand for a hydrogen atom, a lower alkyl group, or, they form, together with the adjacent nitrogen atom, a 5- or 6-membered heterocyclic ring, n denotes an integer of 2 to 4 and X stands for a halogen atom or a salt thereof, which comprises allowing a compound of the formula:

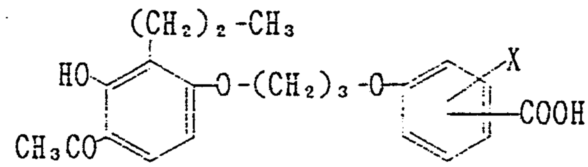

wherein X is of the same meaning as defined above or a salt thereof to react with a reactive derivative of the formula:

$$HO-(CH_2)_n N \begin{matrix} R^1 \\ R^2 \end{matrix}$$

wherein $R^1$, $R^2$ and n are of the same meaning as defined above.

10. A method for producing a benzoic acid derivative of the formula:

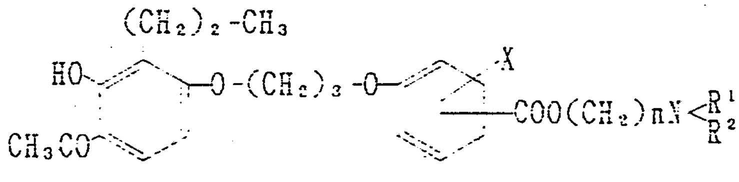

wherein $R^1$ and $R^2$ independently stand for a hydrogen atom, a lower alkyl group, or, they form, together with the adjacent nitrogen atom, a 5- or 6-membered heterocyclic ring, n denotes an integer of 2 to 4 and X stands for a halogen atom or a salt thereof, which comprises allowing a reactive derivative of a compound of the formula:

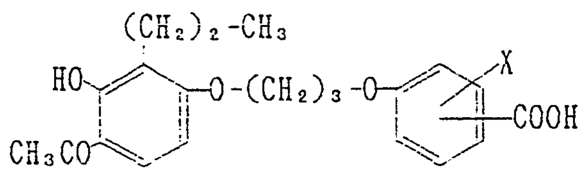

wherein X is of the same meaning as defined above to react with a reactive derivative of the formula:

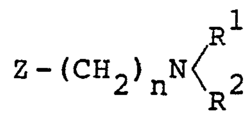

wherein $R^1$, $R^2$ and n are of the same meaning as defined above and Z stands for a leaving group when the compound is esterified.

## A U S T R I A N   C L A I M S

1. A method for producing a benzoic acid derivative of the formula:

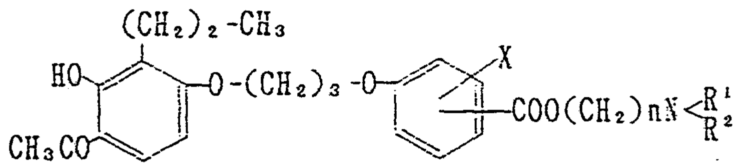

wherein $R^1$ and $R^2$ independently stand for a hydrogen atom, a lower alkyl group, or, they form, together with the adjacent nitrogen atom, a 5- or 6-membered heterocyclic ring, n denotes an integer of 2 to 4 and X stands for a halogen atom or a salt thereof, which comprises allowing a reactive derivative of a compound of the formula:

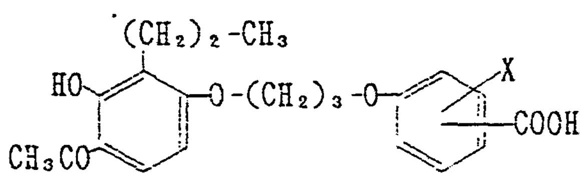

wherein X is of the same meaning as defined above to react with a reactive derivative of the formula:

$$HO-(CH_2)_nN\langle^{R^1}_{R^2}$$

wherein $R^1$, $R^2$ and n are of the same meaning as defined above.

2. A method as claimed in Claim 1, wherein $R^1$ and $R^2$ are lower alkyl groups.

3. A method as claimed in Claim 1, wherein $R^1$ and $R^2$ form, together with the adjacent nitrogen atom, a 6-membered heterocyclic ring.

4. A method as claimed in Claim 1, wherein n is 2.

5. A method as claimed in Claim 1, wherein

n is 3.

6. A method as claimed in Claim 1, wherein X is bromine.

7. A method for producing a benzoic acid derivative of the formula:

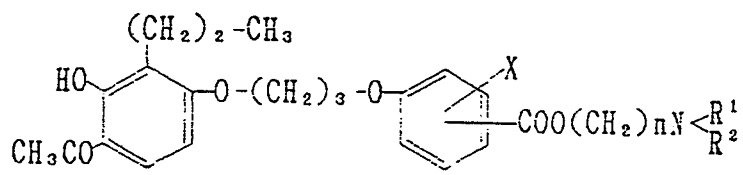

wherein $R^1$ and $R^2$ independently stand for a hydrogen atom, a lower alkyl group, or, they form, together with the adjacent nitrogen atom, a 5- or 6-membered heterocyclic ring, n denotes an integer of 2 to 4 and X stands for a halogen atom or a salt thereof, which comprises allowing a compound of the formula:

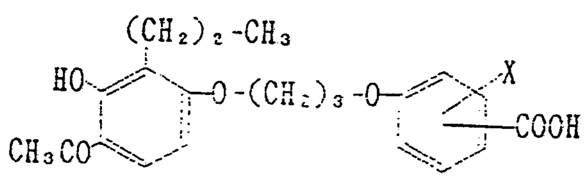

wherein X is of the same meaning as defined above or a salt thereof to react with a reactive derivative of the formula:

$$Z-(CH_2)_nN<^{R^1}_{R^2}$$

wherein $R^1$, $R^2$ and n are of the same meaning as defined above and Z stands for a leaving group when the compound is esterified.

8. A method as claimed in Claim 7, wherein $R^1$ and $R^2$ are lower alkyl groups.

9. A method as claimed in Claim 7, wherein $R^1$ and $R^2$ form, together with the adjacent nitrogen atom, a 6-membered heterocyclic ring.

10. A method as claimed in Claim 7, wherein X is bromine.